# EUROPEAN PATENT SPECIFICATION

(11) **EP 0 796 102 B1**
(45) Date of publication and mention of the grant of the patent: **27.03.2002**
(21) Application number: 95941821.1
(22) Date of filing: 29.12.1995
(51) Int. Cl.: A61K 35/64, A61K 33/38, A61K 33/24

(54) **A COMPOSITION FOR TREATING ENDOMETRIOSIS, CONTAINING A PRECIOUS METAL SALT AND MEL CUM SALE**
ZUSAMMENSETZUNG ZUR BEHANDLUNG VON ENDOMETRIOSE, WELCHE EIN SALZ EINES EDELMETALLES UND MEL CUM SALE ENTHÄLT
COMPOSITION POUR LE TRAITEMENT DE L'ENDOMETRIOSE CONTENANT UN SEL DE METAL PRECIEUX ET MEL CUM SALE

(30) Priority: 30.12.1994 GB 9426409
(43) Date of publication of application: 24.09.1997
(73) Proprietor: Britannia Health Products Ltd., Redhill, Surrey RH1 6YS (GB)
(72) Inventor: WEST, Marlene, Warranwood, VIC 3134 (AU)
(74) Representative: Killin, Stephen James
(86) International application number: GB9503052
(87) International publication number: WO9620720

(56) References cited:
- NETIEN G.; TRAISNEL M.; VERAIN A. : "MEDICAMENTS HOMEOPATHIQUES " 1986 , PARIS, TECHNIQUE ET DOCUMENTATION.; FR XP000013176 see page 92-94 see page 253-262 see page 399-405

## Description

The present invention relates to a composition for use in the therapeutic treatment of a condition of a female reproductive system. In particular, the present invention relates to a composition for the treatment of endometriosis and associated infertility.

Infertility in women can arise from a number of different diseases or conditions. One such condition is endometriosis, in which tissue more or less perfectly resembling the uterine mucous membrane occurs aberrantly in various locations in the pelvic cavity, especially in ovarian tissue in the form of cysts of various sizes. Patients suffering from endometriosis may be unable to conceive or may conceive and subsequently miscarry early in the pregnancy. The condition can be treated by surgery but, in some patients, surgery may be contra-indicated. Moreover, patients are often reluctant to undergo surgery and there is no guarantee that surgery will help restore fertility. The condition can also be treated by therapy with conventional drugs but there are frequently associated side-effects. The use of progestogens such as dydrogesterone, for example, is often associated with bleeding and breast discomfort, whilst the suppression of normal ovulatory cycles can persist for many months after treatment has ended. The use of drugs like danazol and gestrinone, which are structurally related to testosterone, can produce androgenic side-effects, such as acne, hirsuitism and deepening of the voice, as well as causing water-retention in some patients. The use of gonadarelin analogues such as goserelin and buserelin can produce menopause-like symptoms, including hot flushes, increased sweating and changes in libido. There is a need, therefore, for a simple but effective treatment for endometriosis, which does not require recourse to surgery and does not have adverse side-effects, and which allows a patient to lead a normal active life during treatment.

Accordingly, in a first aspect of the invention, there is provided a composition comprising a precious metal salt and *met cum sale*. Advantageously, the inventive composition can be used to treat endometriosis and can help to cure associated infertility. Preferably, the precious metal salt is a double salt, for example comprising an alkaline metal ion, a precious metal cation and a primary acid anion. The precious metal may be gold, silver or platinum but is preferably gold or platinum, most preferably gold. In an embodiment of the invention, the precious metal salt is in the form of the double chloride of gold and sodium, *aurum muriaticum natronatum.* In a preferred embodiment of the present invention, equal volumes of *aurum muriaticum* *natronatum* and *mel cum sale* are mixed, each at a potency of 30c prior to mixing.

In another embodiment of the invention, the composition further comprises an extract from a nut-bearing tree. The extract may be obtained from any nut-bearing tree and can be derived either from the nut of the tree or from the tree itself. In a preferred embodiment, the tree from which the extract is obtained belongs to the genus *Aesculus,* the winter or Italian oak. Preferably, the extract is obtained from a tree of the species *Aesculus hippocastanum*, and most preferably from the nut of the horsechestnut tree, *Aesculus hippocastanum.*

In a further embodiment, the composition also comprises an inorganic acid. In an especially preferred embodiment, the acid is phosphoric acid, *acidum phosphoricum.* Phosphoric acid not only acts as a flavouring agent and adds bulk to the mixture but can also have a beneficial effect in the treatment of endometriosis and can assist in restoring fertility. In a further embodiment, the composition also comprises an analgesic substance. In this last embodiment, the analgesic substance relieves pain and helps to relax the patient, thus allowing the other ingredients of the composition to act more effectively in treating the condition. Preferably, the analgesic substance is a natural plant extract, such as an extract from a plant belonging to the genus *Alchemilla.* Most preferably, the analgesic substance is obtained from Lady's Mantle, *Alchemilla vulgaris.* In another embodiment, the composition further comprises an extract from White Ash, *Fraxinus americana.* A particularly preferred composition according to the first aspect of the present invention comprises *aurum muriaticum natronatum, mel cum sale, acidum phosphoricum*, an extract from *Aesculus hippocastanum,* an extract from *Alchemilla vulgaris,* and an extract from *Fraxinus americana.* Another preferred composition is formable by mixing in substantially the following potencies and amounts: *aurum muriaticum natronatum* (30c, 20 parts by volume), *mel cum sale* (30c, 2.5 parts by volume), *acidum phosphoricum* (6c, 2.5 parts by volume), *Aesculus hippocastanum* (3c, 10 parts by volume), *Alchemilla vulgaris* (3c, 10 parts by volume) and *Fraxinus americana* (3c, 5 parts by volume).

In any of the aforementioned compositions according to the invention in its first aspect, each of the active components may be present at a potency of between Ox and Cm, preferably 6x and 50m, more preferably 12x and 10m, most preferably 9c and 1m and, in particular, between 12c and 200c.

In a second aspect of the invention, there is provided a composition for treating a condition of a female reproductive system, such as infertility or endometriosis. In a third aspect of the invention, there is provided the use of a composition according to the invention in either its first or second aspect for the manufacture of a medicament for therapeutic treatment of a condition of a female reproductive system. In the invention according to either its second or third aspect, the condition to be treated can be any condition associated with any organ of the female reproductive system, especially any uterine, ovarian, cervical or fallopian condition which may restrict or prevent fertility. It is particularly envisaged that the invention in either its second or third aspect may be used in the treatment of infertility in a patient that is caused by or results from endometriosis.

In order that the invention may be better understood, a number of specific embodiments thereof will now be described by way of illustration only.

In an experimental trial, five patients, who had been diagnosed as suffering from endometriosis and who were unable to conceive, were treated with a composition comprising the following ingredients.

| **Ingredient** | **Potency** | **Volume** |
|---|---|---|
| *Aurum muriaticum natronatum* | *30c* | *10 drops* |
| *Mel cum sale* | *30c* | *10 drops* |

Each ingredient was prepared as a separate stock solution at a homoeopathic potency of 30c and succussed 30 times, in the manner described in standard homoeopathic texts, such as Hahnemann's *Organon*, obtainable in English translation from, *inter alia*, *B.* Jain Publishers, Calcutta, India, or *The Science of Homoeopathy* by George Vithoulkas, Thorsons Publishers, 1986. The ingredients can be readily obtained from any reputable homoeopathic pharmacy, such as Ainsworths Homoeopathic Pharmacy, 38 New Cavendish Street, London W1M 7LH. The amounts of each stock solution stated above were mixed with 5 ml of medicinal alcohol and made up to 30 ml with distilled water. Alternatively, each stock solution can be separately added to 5 ml of alcohol and made up to 30 ml with distilled water to give two separate medications which are administered concurrently. Patients received ten drops of the diluted ingredients three times daily and were instructed to avoid taking coffee or peppermints at the same time. Patients were also advised to store the medication away from direct sunlight or microwave ovens and from strong-smelling substances, such as camphor.

### CASE STUDY I

Patient A consulted a gynaecologist for severe period pains and clotting. She was advised that both fallopian tubes were blocked with cysts and adhesions and was prescribed Dupheston over a period of six months. (Dupheston is a registered trade mark.) Thereafter, Patient A was given a laparoscopy and endometriosis was diagnosed. Danizol was prescribed over a period of several months. (Danizol is a registered trade mark.) After treatment with Danizol, Patient A sought advice from a homoeopathic specialist and started treatment with the inventive composition. In the same year, she had an ectopic pregnancy and lost her right fallopian tube. Although her left tube was still partially blocked with scar tissue, she was advised by her gynaecologist that the endometriosis had cleared and that she was eligible for *in vitro* fertilization. Patient A now has twin boys.

### CASE STUDY II

Patient B suffered severe depression following an abortion and was treated homoeopathically for stress-related problems for a number of years. When she finally married, she experienced difficulty in conceiving. Following a laparoscopy, endometriosis was diagnosed and Patient B was prescribed Danizol. Patient B was unhappy with the treatment and sought alternative advice. She was treated with the inventive composition and within one year had conceived. Patient B now has a three year old son.

### CASE STUDY III

Patient C had been trying to conceive for some time. Her gynaecologist performed a laparoscopy and diagnosed endometriosis. Patient C was reluctant to take Danizol because of a previous kidney problem and sought homoeopathic advice. After four months treatment with the inventive composition, Patient C had another laparoscopy which revealed no further signs of endometriosis were present.

### CASE STUDY IV

Patient D had been trying to conceive for eighteen months following a termination of pregnancy some four years previously. A laparoscopy was performed and endometriosis diagnosed. Patient D decided against treatment with Danizol and opted for a homoeopathic approach instead. After two months treatment with the inventive composition, Patient D became pregnant.

### CASE STUDY V

Patient E was diagnosed as having endometriosis. Her menstrual cycle was regular and she had been taking Clomid. (Clomid is a registered trade mark.) She conceived and miscarried at two weeks. Over a period of years, she miscarried seven times, always at six weeks gestation. Her periods were painful and clotted. Within one month of commencing treatment with the inventive composition, Patient E became pregnant. Treatment was continued throughout the pregnancy until the birth of her baby.

Thus, within one month to a year of commencing treatment with the inventive composition, all of the endometriosis patients in the clinical trial group claimed a measure of relief from their symptoms. Subsequently, all five patients went on to make a full recovery and four had successful pregnancies.

In a further example of the invention, the composition comprises *aurum muriaticum natronatum* (30c, 10 drops), *mel cum sale* (30c, 10 drops), *Aesculus hippocastanum* (mother tincture, 1 ml), *acidum phosphoricum* (6c, 1 ml), *Alchemilla vulgaris* (mother tincture, 1 ml) and *Fraxinus americana* (mother tincture, 1 ml). All the ingredients are mixed together, added to 5 ml of alcohol and the final volume of the resultant solution made up to 50 ml with distilled water. Dosage is as described above.

In another formulation according to the invention, the composition comprises *aurum muriaticum natronatum* (30c, 15ml) and *mel cum sale (30c,* 15ml). Both ingredients are mixed to give a total volume of 30 ml. Dosage is 10 drops to be taken twice daily. In a further formulation according to the invention, the composition comprises *aurum muriaticum natronatum* (30c, 20 ml), *mel cum sale* (30c, 2.5 ml), *Aesculus hippocastanum* (3c, 10 ml), *Alchemilla vulgaris* (3c, 10 ml), *acidum phosphoricum* (6c, 2.5ml) and *Fraxinus* *americana* (3c, 5ml). All the ingredients are mixed to give a total volume of 50 ml. Dosage is 10 drops to be taken twice daily.

In alternative compositions according to the invention, the *Aesculus hippocastanum, Fraxinus americana*, *acidum phosphoricum* or *Alchemilla vulgaris* may be omitted, either singly or in any combination. In all of the inventive compositions, the amount and potency of each ingredient used to prepare the composition may be varied within conventional homoeopathic ranges. In particular, the amount of *Aesculus hippocastanum, Fraxinus americana, acidum phosphoricum* or *Alchemilla vulgaris* may be present in any amount tolerated by the patient between 1 drop and 2ml, preferably 1 drop and 20 drops, most preferably 10 drops and 20 drops and especially in an amount of 10 drops. At the same time, the *aurum muriaticum natronatum* and *mel cum sale* may be present in any amount tolerated by the patient between 1 drop and 2 ml, preferably 1 drop and 20 drops, most preferably 10 drops and 20 drops and especially in an amount of 10 drops. Similarly, any of the ingredients used to prepare the inventive composition may be used in any potency tolerated by the patient, including 0-6x, 12x, 9c, 12c, 30c, 200c, 1m, 10m, 50m and cm.

The recommended dosages and treatment regimens employed using the inventive compositions may be adapted in accordance with conventional homeopathic or herbalist practices. In particular, the inventive compositions may be used undiluted or may be further diluted before administration, for example, by taking 10 drops of the undiluted composition, adding it to 5 ml of alcohol and making up to 30 ml or 50 ml with distilled water. In this last example, 10 drops of the diluted composition may then be administered twice or three times daily to a patient, as required.

## Claims

1. A composition comprising a precious metal salt and *mel cum sale.*

2. A composition as claimed in claim 1, wherein the precious metal salt is a double salt.

3. A composition as claimed in claim 2, wherein the double salt comprises an alkali metal cation, a precious metal cation and a primary acid anion.

4. A composition as claimed in claim 3, wherein the precious metal is gold or platinum.

5. A composition as claimed in claim 4, wherein the precious metal is gold.

6. A composition as claimed in claim 5, wherein the precious metal salt is *aurum muriaticum natronatum*.

7. A composition as claimed in claim 6, formable by mixing substantially equal volumes of *aurum muriaticum natronatum* and *mel cum sale,* each at a potency of 30c prior to mixing.

8. A composition as claimed in any one of the preceding claims, further comprising an extract from a nut-bearing tree.

9. A composition as claimed in claim 8, wherein the extract is obtained from a species of tree belonging to the genus *Aesculus.*

10. A composition as claimed in claim 9, wherein the extract is obtained from *Aesculus hippocastanum.*

11. A composition as claimed in claim 10, wherein the extract is obtained from a nut.

12. A composition as claimed in any one of the preceding claims, further comprising an inorganic acid.

13. A composition as claimed in claim 12, wherein the inorganic acid is *acidum phosphoricum.*

14. A composition as claimed in any one of the preceding claims, further comprising an analgesic substance.

15. A composition as claimed in claim 14, wherein the analgesic substance is a natural plant extract.

16. A composition as claimed in claim 15, wherein the natural plant extract is obtained from a species of plant belonging to the genus *Alchemilla.*

17. A composition as claimed in claim 16, wherein the extract is obtained from *Alchemilla vulgaris.*

18. A composition as claimed in any one of the preceding claims, further comprising an extract from *Fraxinus americana.*

19. A composition comprising *aurum muriaticum natronatum, mel cum sale, acidum phosphoricum*, an extract from *Aesculus hippocastanum*, an extract from *Alchemilla vulgaris,* and an extract from *Fraxinus americana.*

20. A composition as claimed in claim 19, formable by mixing in substantially the following potencies and amounts: *aurum muriaticum natronatum* (30c, 20 parts by volume), *mel cum sale* (30c, 2.5 parts by volume), *acidum* *phosphoricum* (6c, 2.5 parts by volume), *Aesculus hippocastanum* (3c, 10 parts by volume), *Alchemilla vulgaris* (3c, 10 parts by volume) and *Fraxinus americana* (3c, 5 parts by volume).

21. A composition as claimed in any one of claims 1-6 and 8-19, wherein each component is present at a potency of between Ox and Cm.

22. A composition as claimed in claim 21, wherein each component is present at a potency of between 6x and 50m.

23. A composition as claimed in claim 22, wherein each component is present at a potency of between 12x and 10m.

24. A composition as claimed in claim 23, wherein each component is present at a potency of between 9c and 1m.

25. A composition as claimed in claim 24, wherein each component is present at a potency of between 12c and 200c.

26. A composition as claimed in any one of the preceding claims, for therapeutic treatment of a condition of a female reproductive system.

27. A composition as claimed in claim 26, wherein the condition causes infertility.

28. A composition as claimed in claim 27, wherein the condition is endometriosis.

29. Use of a composition as claimed in any one of claims 1-28 for use in a therapeutic treatment.

30. Use as claimed in claim 29, wherein the therapeutic treatment is treatment of a condition of a female reproductive system.

## Patentansprüche

1. Eine Zusammensetzung, die ein Salz eines Edelmetalls und *mel cum sale* (Honig mit Salz) enthält.

2. Eine Zusammensetzung gemäß Anspruch 1, bei der das salz eines Edelmetalls ein Doppelsalz ist.

3. Eine Zusammensetzung gemäß Anspruch 2, bei der das Doppelsalz ein Alkalimetall-Kation, ein Kation eines Edelmetalls und ein Anion einer primären Säure enthält.

4. Eine Zusammensetzung gemäß Anspruch 3, bei der das Edelmetall aus Gold oder Platin besteht.

5. Eine Zusammensetzung gemäß Anspruch 4, bei der das Edelmetall aus Gold besteht.

6. Eine Zusammensetzung gemäß Anspruch 5, bei der das Salz des Edelmetalls aus *aurum muriaticum natronatum* (Natrium-Chloroaurat) besteht.

7. Eine Zusammensetzung gemäß Anspruch 6, herstellbar durch das vermischen von im wesentlichen gleichen Bestandteilen an *aurum muriaticum natronatum* (Natrium-Chloroaurat) und *mel cum sale* (Honig mit Salz), wobei jede dieser Substanzen vor dem Mischen eine Potenz von 30c aufweist.

8. Eine Zusammensetzung gemäß einem der vorangehenden Ansprüche, die außerdem einen Extrakt von einem nußtragenden Baum enthält.

9. Eine Zusammensetzung gemäß Anspruch 8, bei der der Extrakt von einer Baumspezies stammt, die zur Gattung *Aesculus* (Kastanie) gehört.

10. Eine Zusammensetzung gemäß Anspruch 9, bei der der Extrakt von *Aesculus hippocastanum* (Roßkastanie) stammt.

11. Eine Zusammensetzung gemäß Anspruch 10, bei der der Extrakt von einer Nuß stammt.

12. Eine Zusammensetzung gemäß einem der vorangehenden Ansprüche, die außerdem eine anorganische Säure enthält.

13. Eine zusammensetzung gemäß Anspruch 12, bei der die anorganische Säure aus *acidum phosphoricum* (Phosphorsäure) besteht.

14. Eine Zusammensetzung gemäß einem der vorangehenden Ansprüche, die außerdem eine analgetische Substanz enthält.

15. Eine Zusammensetzung gemäß Anspruch 14, bei der die analgetische Substanz aus einem natürlichen Pflanzenextrakt besteht.

16. Eine Zusammensetzung gemäß Anspruch 15, bei der der natürliche Pflanzenextrakt von einer Pflanzenspezies stammt, die zur Gattung *Alchemilla* (Frauenmantel) gehört.

17. Eine Zusammensetzung gemäß Anspruch 16, bei der der Extrakt von *Alchemilla vulgaris* (Wiesen-Frauenmantel) stammt.

18. Eine Zusammensetzung gemäß einem der vorangehenden Ansprüche, die außerdem einen Extrakt von *Fraxinus americana* (Weiße Asche) enthält.

19. Eine Zusammensetzung, die *aurum muriaticum natronatum* (Natrium-Chloroaurat), *mel cum sale* (Honig mit Salz), *acidum phosphoricum* (Phosphorsäure), einen Extrakt von *Aesculus hippocastanum* (Roßkastanie), einen Extrakt von *Alchemilla vulgaris* (Wiesen-Frauenmantel) und einen Extrakt *von Fraxinus americana* (Weiße Asche) enthält.

20. Eine Zusammensetzung gemäß Anspruch 19, herstellbar durch das Vermischen von im wesentlichen den folgenden Potenzen und Mengen: *aurum muriaticum natronatum* (Natrium-Chloroaurat, 30c, 20 Teile pro volumen), *mel cum sale* (Honig mit Salz, 30c, 2,5 Teile pro Volumen), *acidum phosphoricum* (phosphorsäure, 6c, 2,5 Teile pro Volumen), *Aesculus hippocastanum* (Roßkastanie, 3c, 10 Teile pro Volumen), *Alchemilla vulgaris* (Wiesen-Frauenmantel, 3c, 10 Teile pro Volumen) und *Fraxinus americana* (Weiße Asche, 3c, 5 Teile pro Volumen.

21. Eine Zusammensetzung gemäß einem der Ansprüche 1-6 und 8-19, in der jede Komponente in einer Potenz zwischen Ox und Cm enthalten ist.

22. Eine Zusammensetzung gemäß Anspruch 21, in der jede Komponente in einer Potenz zwischen 6x und 50m enthalten ist.

23. Eine Zusammensetzung gemäß Anspruch 22, in der jede Komponente in einer Potenz zwischen 12x und 10m enthalten ist.

24. Eine Zusammensetzung gemäß Anspruch 23, in der jede Komponente in einer Potenz zwischen 9c und 1m enthalten ist.

25. Eine Zusammensetzung gemäß Anspruch 24, in der jede Komponente in einer Potenz zwischen 12c und 200c enthalten ist.

26. Eine Zusammensetzung gemäß einem der vorangehenden Ansprüche, zur therapeutischen Behandlung eines Zustandes eines weiblichen Reproduktionssystems.

27. Eine Zusammensetzung gemäß Anspruch 26, bei der der Zustand Unfruchtbarkeit hervorruft.

28. Eine Zusammensetzung gemäß Anspruch 26, bei der der zustand derjenige der Endometriose ist.

29. Verwendung einer Zusammensetzung gemäß einem der Ansprüche 1-28 für die Anwendung bei einer therapeutischen Behandlung.

30. Verwendung gemäß Anspruch 29, wobei die therapeutische Behandlung eine Behandlung eines weiblichen Reproduktionssystems ist.

## Revendications

1. Une composition contenant un sel de métal précieux et du *mel cum sale.*

2. Une composition selon la revendication 1, dans laquelle le sel de métaux précieux est un sel double.

3. Une composition selon la revendication 2, dans laquelle le sel double contient un cation de métal alcalin, un cation de métal précieux et un anion d'acide primaire.

4. Une composition selon la revendication 3, dans laquelle le métal précieux est l'or ou le platine.

5. Une composition selon la revendication 4, dans laquelle le métal précieux est l'or.

6. Une composition selon la revendication 5, dans laquelle le sel de métal précieux et *l'aurum muriaticum natronatum.*

7. Une composition selon la revendication 3, qui peut être réalisée par mélangeage de volumes partiquement égaux d'*aurum muriaticum natronatum* et de *mel cum sale,* chacun à une puissance de 30c avant mélangeage.

8. Une composition selon l'une quelconque des revendications précédentes, contenant au surplus un extrait d'un arbre porteur de noix.

9. Une composition selon la revendication 8, dans laquelle l'extrait est obtenu d'une espèce d'un arbre appartenant à la famille des *Aesculus.*

10. Une composition selon la revendication 9, dans laquelle l'extrait est obtenu à partir d'*Aesculus hippocastanum.*

11. Une composition selon la revendication 10, dans laquelle l'extrait est obtenu à partir d'une noix.

12. Une composition selon l'une quelconque des revendications précédentes, contenant au surplus un acide minéral.

13. Une composition selon la revendication 12, dans laquelle l'acide minéral est l'*acidum phosphoricum*.

14. Une composition selon l'une quelconque des revendications précédentes, contenant au surplus une substance analgésique.

15. Une composition selon la revendication 14, dans laquelle la substance analgésique est un extrait naturel de plante.

16. Une composition selon la revendication 15, dans laquelle l'extrait naturel de plante est obtenu à partir d'une espèce d'une plante appartenant à la famille des *Alchemilla.*

17. Une composition selon la revendication 16, dans laquelle l'extrait est obtenu à partir d'*Alchemilla vulgaris.*

18. Une composition selon l'une quelconque des revendications précédentes, contenant au surplus un extrait de *Fraxinus americana*.

19. Une composition comprenant de l'*aurum muriaticum natronatum*, du *mel cum sale,* de l'*acidum phosphoricum*, un extrait d'*Aesculus hippocastanum*, un extrait d'*Alchemilla vulgaris* et un extrait de *Fraxinus americana*.

20. Une composition selon la revendication 19, qui peut être réalisée par mélangeage, dans les puissances et quantités suivantes : *aurum muriaticum natronatum* (30c, 20 parties en volume), *mel cum sale* (30c, 2,5 parties en volumes), *acidum phosphoricum* (6c, 2,5 parties en volume), *Aesculus hippocastanum* (3c, 2,5 parties en volume), *Alchemilla vulgaris* (3c, 10 parties en volume) et *Fraxinus americana* (3c, 5 parties en volumes).

21. Une composition selon l'une quelconque des revendications 1-6 et 8-19, dans laquelle chaque composant est présent à raison d'une puissance comprise entre Ox et Cm.

22. Une composition selon la revendication 21, dans laquelle chaque composant est présent à raison d'une puissance comprise entre 6x et 50m.

23. Une composition selon la revendication 22, dans laquelle chaque composant est présent à raison d'une puissance comprise entre 12x et 10m.

24. Une composition selon la revendication 23, dans laquelle chaque composant est présent à raison d'une puissance comprise entre 9c et 1m.

25. Une composition selon la revendication 24, dans laquelle chaque composant est présent à raison d'une puissance comprise entre 12c et 200c.

26. Une composition selon l'une quelconque des revendications précédentes, destinée au traitement thérapeutique d'un état du système reproducteur féminin.

27. Une composition selon la revendication 26, dans laquelle l'état provoque l'infertilité.

28. Une composition selon la revendication 27, dans laquelle l'état est l'endométriose.

29. Utilisation de l'une des compositions selon l'une quelconque des revendications 1 à 28 dans un traitement thérapeutique.

30. Utilisation selon la revendication 29, dans laquelle le traitement thérapeutique est un traitement d'un état du système reproducteur féminin.
